# EUROPEAN PATENT APPLICATION

(11) **EP 4 796 563 A1**
(43) Date of publication of application: **26.08.2026**
(21) Application number: 24881170.5
(22) Date of filing: 08.08.2024
(51) Int. Cl.: C07K 16/28, C12N 15/85, C12N 15/13

(54) **FULLY HUMAN TSH RECEPTOR BLOCKING MONOCLONAL ANTIBODY AND PREPARATION THEREFOR AND USE THEREOF**

(30) Priority: 27.10.2023 CN 202311409632
(71) Applicant: SHANGHAI NINTH PEOPLE'S HOSPITAL, SHANGHAI JIAOTONG UNIVERSITY SCHOOL OF MEDICINE, Huangpu District Shanghai 200011 (CN)
(72) Inventor: SONG, Huaidong, Shanghai 200011 (CN); LI, Rui, Shanghai 200011 (CN); ZHANG, Caoxu, Shanghai 200011 (CN)
(74) Representative: Bayramoglu et al.
(86) International application number: PCT/CN2024/110559
(87) International publication number: WO 2025/086801

(57) **Abstract**

The present disclosure provides a fully human thyroid-stimulating hormone receptor (TSHR)-blocking monoclonal antibody for treating hyperthyroidism, and a preparation method and use thereof. The preparation method includes the following steps: isolating plasma cells and memory single B cells that specifically recognize TSHR from peripheral blood of a patient with a high TSHR-blocking antibody (TBAb) titer by flow cytometry; cloning antibody genes for heavy and light chains *in vitro,* and conducting recombinant expression; and screening and verifying antibodies based on properties using hTSHR-CHO cells, so as to obtain the blocking monoclonal antibody specifically targeting human TSHR. The fully human TSHR-blocking monoclonal antibody can specifically bind to TSHR and exhibits excellent inhibitory activity in both *in vitro* and *in vivo* experiments. Therefore, the fully human TSHR-blocking monoclonal antibody demonstrates promising application prospects in the development of treatments for Graves' disease (GD).

## Description

### TECHNICAL FIELD

The present disclosure belongs to the fields of monoclonal antibody technology and human antibody-based drugs, and relates to a fully human thyroid-stimulating hormone receptor (TSHR)-blocking monoclonal antibody, and preparation and use thereof.

### BACKGROUND TECHNOLOGY

Graves' disease (GD) is an organ-specific autoimmune disorder that is characterized by increased secretion of thyroid hormones and results from the combined effects of genetic and environmental factors. GD is the most common cause of hyperthyroidism. GD affects a large population. The incidence rate of GD in the general population is approximately 0.2% to 2%. Similar to other autoimmune diseases, GD predominantly affects women of childbearing age. The incidence rate of GD is approximately 5 times to 10 times higher in women than in men. In recent years, with changes in living environments, the occurrence of hyperthyroidism has continuously increased. If not controlled in a timely manner, the symptoms of hyperthyroidism can affect the heart, leading to arrhythmias or heart failure. In women of childbearing age, hyperthyroidism may cause menstrual disorders, trouble conceiving, miscarriages, etc. In addition, hyperthyroidism can often induce mental abnormalities such as tension, anxiety, and irritability, which affect the learning and daily life of patients and may even trigger mental disorders in severe cases. The current treatments for GD mainly include medication, radioactive iodine, and surgical therapy. The medication has a long course of treatment to which patients can hardly adhere, and is associated with a high recurrence rate. About 60% to 70% of GD patients cured by medication experience relapse. Clinically, the treatments for GD have not seen substantial changes for many years, and the therapeutic options remain anti-thyroid drugs (ATDs), radioactive iodine, and surgery. ATD therapy for GD has a history of nearly 70 years. Outside the United States, physicians worldwide prefer ATD as the first-line treatment for GD. However, after patients with hyperthyroidism receive a standardized and systematic treatment, only a portion of the patients can be cured, and there are considerable side effects. A substantial proportion of the patients will relapse after a period of time. When relapse occurs, the condition often worsens, and radioactive iodine or surgical therapy is generally required, which significantly exacerbates the financial and psychological burdens on patients. Further, radioactive iodine-131 therapy is prone to causing permanent hypothyroidism. The complications of surgical therapy should also not be overlooked. The current treatment dilemma for GD means that there is an urgent clinical need to develop improved alternative drugs.

TSHR antibodies (TRAb) are the characteristic antibodies of GD. TRAb refers to a group of polyclonal antibodies that act on different binding sites of the TSHR and can be classified into TSHR-stimulating antibodies (TSAb), TSHR-blocking antibodies (TBAb), and neutral TSHR antibodies. TSAb binds to TSHR to induce a biological effect similar to thyroid-stimulating hormone (TSH), which is the direct cause of GD. TBAb binds to TSH and blocks the binding of TSH to TSHR, thereby inhibiting thyroid hyperplasia and thyroid hormone production. Both stimulating and blocking antibodies coexist in GD patients, and the ultimate outcome of thyroid function depends on which antibody predominates. As a result, the exogenous supplementation of a blocking antibody for binding to TSHR at a specified dose to ameliorate the pathophysiological effects of autoantibodies has become a new and effective treatment strategy for GD.

Humanized monoclonal antibodies can be divided into human-mouse chimeric monoclonal antibodies and fully human monoclonal antibodies. Although the human-mouse chimeric monoclonal antibodies reduce the proportion of other non-human components to some extent, all non-human components cannot be completely eliminated. Moreover, the engineering of an antibody may reduce the affinity and original biological activity of the antibody to some degree. The fully human monoclonal antibodies are produced as follows: Antibody genes are directly amplified from isolated single plasma cells or memory B cells to obtain a large number of naturally paired antibody genes for heavy and light chains. These paired antibody genes for heavy and light chains are then expressed, and antibodies with antigen specificity and neutralizing activity are ultimately identified by screening. This process offers advantages such as high speed, high throughput, and small cell consumption. The fully human antibodies prepared accordingly retain the excellent diversity of genes and the natural pairing of heavy-chain and light-chain variable regions, presenting tremendous superiority. Currently, the preparation of all fully human antibodies against influenza viruses, *Bacillus anthracis,* and *Pneumococcus* is based on this technology.

### CONTENT OF THE INVENTION

In view of the prior art, the present disclosure provides a fully human TSHR-blocking monoclonal antibody for treating hyperthyroidism, a coding sequence for the fully human TSHR-blocking monoclonal antibody, and a vector including the coding sequence.

In the present disclosure, the term "TSHR" refers to a full-length human thyroid-stimulating hormone receptor with an amino acid sequence shown in SEQ ID NO: 5, or a variant or fragment highly homologous to the thyroid-stimulating hormone receptor. Preferably, the variant or fragment has a homology of 70% to 99.9% with the amino acid sequence shown in SEQ ID NO: 5.

The present disclosure provides a fully human TSHR-blocking monoclonal antibody, where the fully human TSHR-blocking monoclonal antibody is capable of binding to TSHR to block binding of TSH to TSHR; an amino acid sequence of the fully human TSHR-blocking monoclonal antibody is shown in SEQ ID NO: 1 or 2; and a nucleotide sequence for the fully human TSHR-blocking monoclonal antibody is shown in SEQ ID NO: 3 or 4.

Preferably, the amino acid sequence of the fully human TSHR-blocking monoclonal antibody has a sequence identity of at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% with SEQ ID NO: 1 or 2; and/or the nucleotide sequence for the fully human TSHR-blocking monoclonal antibody has a sequence identity of at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% with SEQ ID NO: 3 or 4.

Preferably, the amino acid sequence of the fully human TSHR-blocking monoclonal antibody has a sequence identity of at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% with SEQ ID NO: 1 or SEQ ID NO: 2.

Preferably, the nucleotide sequence for the fully human TSHR-blocking monoclonal antibody has a sequence identity of at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% with SEQ ID NO: 3 or SEQ ID NO: 4.

The present disclosure includes a fully human TSHR-blocking monoclonal antibody TBI80. K1-70 in Table 1 is a reported TSHR-blocking monoclonal antibody with a blocking activity. In contrast, the sequences provided by the present disclosure are significantly different from and share no homology with sequences of K1-70.

Preferably, the present disclosure provides a fully human TSHR-blocking monoclonal antibody TBI80.

In the present disclosure, the fully human TSHR-blocking monoclonal antibody is an antagonist of TSH.

In the present disclosure, the fully human TSHR-blocking monoclonal antibody is an antagonist of a TSHR-stimulating antibody.

In the present disclosure, the fully human TSHR-blocking monoclonal antibody includes a V_{H} region, and the V_{H} region includes one or more complementarity determining regions (CDRs) selected from SEQ ID NO: 6 to SEQ ID NO: 8 (as shown in Table 1).

In the present disclosure, the fully human TSHR-blocking monoclonal antibody includes a V_{L} region, and the V_{L} region includes one or more CDRs selected from SEQ ID NO: 9 to SEQ ID NO: 11 (as shown in Table 1).

In the present disclosure, the fully human TSHR-blocking monoclonal antibody includes one or more amino acid sequences substantially homologous to the CDRs.

The fully human TSHR-blocking monoclonal antibody provided by the present disclosure can bind to TSHR, thereby inhibiting signal transduction of the TSHR; inhibit the synthesis and secretion of thyroid hormones, thereby reducing hyperthyroidism caused by various factors; significantly reduce goiters caused by hyperthyroidism or other conditions upon binding to TSHR; and alleviate inflammation, edema, and hyperplasia associated with thyroid-associated ophthalmopathy.

The present disclosure also provides a use of the fully human TSHR-blocking monoclonal antibody described above in preparation of a drug for treating a thyroid-related disorder.

The present disclosure also provides a use of the fully human TSHR-blocking monoclonal antibody described above in preparation of a reagent or a kit for detecting a TSHR antibody.

The present disclosure also provides a use of the fully human TSHR-blocking monoclonal antibody described above in preparation of a reagent or a kit or a product for detecting hyperthyroidism or thyroid-associated ophthalmopathy.

The present disclosure also provides a use of the fully human TSHR-blocking monoclonal antibody described above in preparation of a drug for treating a disease such as hyperthyroidism or thyroid-associated ophthalmopathy; in preparation of a drug for inhibiting thyroid hyperplasia and/or thyroid hormone production; in preparation of a drug for blocking TSHR; or in preparation of a drug for antagonizing an activating effect of TSH on TSHR.

The present disclosure also provides a formulation, a drug, or a pharmaceutical composition, including the fully human TSHR-blocking monoclonal antibody described above.

The present disclosure also provides a use of the formulation, the drug, or the pharmaceutical composition described above in preparation of a formulation, a drug, or a pharmaceutical composition for detecting a TSHR antibody; in preparation of a drug for treating hyperthyroidism or thyroid-associated ophthalmopathy; in preparation of a drug for inhibiting thyroid hyperplasia and/or thyroid hormone production; in preparation of a drug for blocking TSHR; or in preparation of a drug for antagonizing an activating effect of TSH on TSHR.

The present disclosure also provides a use of the fully human TSHR-blocking monoclonal antibody described above in preparation of a formulation, a drug, or a pharmaceutical composition for detecting a TSHR antibody.

Further, the formulation, the drug, or the pharmaceutical composition may further include a physiologically compatible adjuvant. The adjuvant includes a buffer, a diluent, an excipient, a filler, a binder, a wetting agent, a disintegrant, an absorption enhancer, a surfactant, an adsorption carrier, a lubricant, etc.

Further, the formulation, the drug, or the pharmaceutical composition can be prepared into an injection, a sterile powder for injection, a tablet, a pill, a capsule, a lozenge, a spirit, a powder, a granule, a syrup, a solution, a tincture, an aerosol, a dry powder for inhalation, a suppository, etc. The various dosage forms of the formulation, the drug, or the pharmaceutical composition can all be prepared according to conventional methods in the pharmaceutical field.

Further, the formulation, the drug, or the pharmaceutical composition may be introduced into a body such as a muscle, an intradermal tissue, a subcutaneous tissue, a vein, or a mucosal tissue by injection, spraying, nasal instillation, ocular instillation, penetration, absorption, or a physically or chemically mediated method, or may be introduced into a body after being mixed with or encapsulated by another substance.

The present disclosure also provides a reagent, a kit, or a product, including the fully human TSHR-blocking monoclonal antibody. Preferably, the reagent or the kit for detecting hyperthyroidism and thyroid-associated ophthalmopathy includes the fully human TSHR-blocking monoclonal antibody.

The present disclosure also provides a use of the fully human TSHR-blocking monoclonal antibody described above in preparation of a reagent or a kit or a product for detecting hyperthyroidism or thyroid-associated ophthalmopathy.

The present disclosure also provides a use of the reagent or the kit, or the product described above in preparation of a drug for treating hyperthyroidism or thyroid-associated ophthalmopathy; in preparation of a drug for inhibiting thyroid hyperplasia and/or thyroid hormone production; in preparation of a drug for blocking TSHR; or in preparation of a drug for antagonizing an activating effect of TSH on TSHR.

The present disclosure also provides a method for preparing a fully human TSHR-blocking monoclonal antibody, including the following steps:
(1) isolating plasma cells and memory B cells targeting TSHR from peripheral blood of a patient with a high TBAb activity; conducting single-cell RNA extraction and cDNA synthesis, and verifying genes of heavy chains (H), a light chain (λ), and a light chain (κ) amplified from isolated single cells by nested polymerase chain reaction (PCR); and selecting single-cell clones positive for both the heavy chains and the light chains for subsequent cloning;
(2) conducting *in vitro* amplification by nested PCR, and cloning genes of B cell receptor (BCR) heavy and light chains from all single B cells into a heavy-chain expression vector (AbVec-IGHG 1), a light-chain expression vector (AbVec-hlgKappa), and a light-chain expression vector (AbVec-hlgLambda), respectively; and
(3) after heavy-chain and light-chain recombinant plasmids are successfully obtained, subjecting acquired candidate clones to sequencing and alignment analysis to determine numbers of nucleotide and amino acid sequences of candidate antibodies; transfecting the heavy-chain and light-chain recombinant plasmids to express monoclonal antibodies *in vitro;* and further verifying an antigen-binding capacity and an antibody blocking activity to finally obtain an antibody combination specifically targeting a target antigen, which is the fully human TSHR-blocking monoclonal antibody.

The present disclosure also provides a method for treating a thyroid-related disorder in a mammalian subject or in a cell derived from the mammalian subject, including contacting the mammalian subject or the cell with the fully human TSHR-blocking monoclonal antibody described above.

The thyroid-related disorder is selected from hyperthyroidism, thyroid-associated ophthalmopathy (Graves' ophthalmopathy), neonatal hyperthyroidism, human chorionic gonadotropin-induced hyperthyroidism, excessive thyroid activity, thyroid cancer, thyroiditis, and pretibial myxedema.

The present disclosure also provides a method for inhibiting stimulation of TSHR by a TSHR-stimulating antibody in a thyroid of a mammalian subject, including contacting the mammalian subject with the fully human TSHR-blocking monoclonal antibody of the present disclosure.

Preferably, the TSHR-stimulating antibody is prevented from binding to the TSHR.

The present disclosure also provides a method for inhibiting binding of a TSHR-stimulating autoantibody to extrathyroidal TSHR in a mammalian subject, including contacting the mammalian subject with the fully human TSHR-blocking monoclonal antibody described above.

Specifically, the extrathyroidal TSHR is located in a retro-orbital tissue and/or a pretibial tissue of the mammalian subject.

Preferably, the fully human TSHR-blocking monoclonal antibody can block the binding of the TSHR-stimulating autoantibody to the extrathyroidal TSHR.

The present disclosure also provides a method for treating thyroid cancer or metastatic thyroid cancer in a thyroid of a subject or in a thyroid cell derived from the subject, including contacting the cancer cell with the fully human TSHR-blocking monoclonal antibody described above to inhibit a constitutive TSHR activity in the cancer cell.

Preferably, regrowth of a thyroid cancer cell is prevented or delayed.

The present disclosure also provides a method for treating an excessive thyroid activity caused by a constitutive thyroid activity in a subject or in a thyroid cell derived from the subject, including contacting the subject or the thyroid cell with the fully human TSHR-blocking monoclonal antibody described above to inhibit the excessive thyroid activity.

The present disclosure also provides a method for identifying a molecule capable of inhibiting binding of a TSHR-stimulating antibody to TSHR, including providing at least one fully human TSHR-blocking monoclonal antibody described above as a reference.

Preferably, a molecule capable of blocking the binding of the TSHR-stimulating antibody to the TSHR is selected.

The present disclosure also provides a method for identifying a molecule capable of inhibiting binding of TBAb to TSHR, including providing at least one fully human TSHR-blocking monoclonal antibody described above as a reference.

Preferably, a molecule capable of blocking the binding of the TBAb to the TSHR is selected.

Specifically, the thyroid-related disorder is selected from hyperthyroidism, thyroid-associated ophthalmopathy (Graves' ophthalmopathy), neonatal hyperthyroidism, human chorionic gonadotropin-induced hyperthyroidism, excessive thyroid activity, thyroid cancer, thyroiditis, and pretibial myxedema.

Preferably, in the above methods, the subject to be treated is a human.

The method for preparing the fully human TSHR-blocking monoclonal antibody in the present disclosure includes the following steps: isolating plasma cells and memory single B cells that specifically recognize TSHR from peripheral blood of a patient with a high TBAb titer by flow cytometry; cloning antibody genes for heavy and light chains *in vitro,* and conducting recombinant expression; and screening and verifying antibodies based on properties using hTSHR-CHO cells, so as to obtain the blocking monoclonal antibody specifically targeting human TSHR. With the method for preparing the fully human TSHR-blocking monoclonal antibody for treating hyperthyroidism provided in the present disclosure, the target antibody sequence can be obtained within 3 weeks to 1 month.

Further, in the present disclosure, antibody property verification and efficacy evaluation are conducted *in vitro* using hTSHR-CHO cells, and a blocking effect of the antibody is confirmed through an animal experiment.

The present disclosure also provides a nucleotide sequence encoding the fully human TSHR-blocking monoclonal antibody described above, where the nucleotide sequence is one of the following sequences:
(1) a nucleotide sequence shown in SEQ ID NO: 3 or SEQ ID NO: 4;
(2) a nucleotide sequence having a sequence identity of at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% with SEQ ID NO: 3 or SEQ ID NO: 4;
(3) a nucleotide sequence obtained by adding, substituting, deleting, or inserting one or more nucleotides relative to the nucleotide sequence shown in SEQ ID NO: 3 or SEQ ID NO: 4;
(4) a nucleotide sequence capable of hybridizing to the nucleotide sequence defined in the (1), the (2), or the (3) or a full-length complement thereof under stringent conditions; or
(5) a nucleotide sequence different from the nucleotide sequence defined in the (1), the (2), the (3), or the (4) due to genetic code degeneracy.

The nucleotide sequence or a portion thereof encodes an antibody V_{H} domain and an antibody V_{L} domain or CDRs selected from SEQ ID NO: 6 to SEQ ID NO: 11 (as shown in Table 1).
Amino acid sequence shown in SEQ ID NO: 1 
Val His Ser Glu Val Gln Leu Val Glu Ser Gly Gly Gly Leu Val Gln Pro Gly Gly Ser Leu Arg
Leu Ser Cys Val Ala Ser Gly Phe Asp Phe Asn Gln Tyr Ser Met Asn Trp Val Arg Gln Ala Pro Gly
Lys Gly Leu Glu Trp Leu Ser Tyr Ile Ser Ser Ser Ala Ala Thr Val Phe Tyr Ala Asp Ser Val Gln Gly
Arg Leu Thr Ile Ser Arg Asp Asn Gly Lys Asn Ser Leu Phe Leu Gln Met Asn Ser Leu Arg Val Glu
Asp Thr Ala Val Tyr Tyr Cys Val Arg Glu Ala Val Ile Val Asp Gly Met Pro Phe Glu Tyr Trp Gly
Gln Gly Ala Leu Val Thr Val Ser Ser
 
(VHSEVQLVESGGGLVQPGGSLRLSCVASGFDFNQYSMNWVRQAPGKGLEWLSYIS
SSAATVFYADSVQGRLTISRDNGKNSLFLQMNSLRVEDTAVYYCVREAVIVDGMPFEYW
GQGALVTVSS).
Amino acid sequence shown in SEQ ID NO: 2 
Val His Ser Glu Ile Val Leu Thr Gln Ser Pro Gly Thr Leu Ser Leu Ser Pro Gly Glu Arg Ala
Thr Leu Ser Cys Arg Ala Ser Gln Ser Val Thr Ser Ser Gln Leu Ala Trp Tyr Gln Gln Lys Pro Gly
Gln Ala Pro Arg Leu Leu Ile Tyr Gly Glu Ser Ser Arg Ala Thr Gly Ile Pro Asp Arg Phe Ser Gly
Arg Gly Ser Gly Thr Asp Phe Ala Leu Thr Ile Ser Arg Leu Glu Pro Glu Asp Phe Ala Val Tyr Cys
Cys Gln Gln Tyr Gly Thr Ser Ile Ala Phe Gly Gly Gly Thr Lys Val Glu Ile Lys
 
(VHSEIVLTQSPGTLSLSPGERATLSCRASQSVTSSQLAWYQQKPGQAPRLLIYGESSR
ATGIPDRFSGRGSGTDFALTISRLEPEDFAVYCCQQYGTSIAFGGGTKVEIK).
Nucleotide sequence shown in SEQ ID NO: 3 
GTACATTCTGAGGTGCAGCTGGTGGAGTCCGGTGGAGGCTTGGTTCAACCGGGG
GGGTCCCTGAGACTCTCCTGTGTAGCCTCTGGATTCGACTTCAACCAATATAGCATGA
ACTGGGTCCGCCAGGCTCCAGGGAAGGGACTGGAGTGGCTTTCATACATCAGTAGCA
GCGCTGCAACCGTGTTCTACGCAGACTCTGTGCAGGGCCGACTCACCATCTCCAGAG
ACAATGGCAAGAATTCACTCTTTCTGCAGATGAACAGCCTGAGAGTCGAGGACACGG
CTGTCTATTACTGTGTCAGAGAAGCTGTTATAGTGGATGGCATGCCGTTTGAATACTGG
GGCCAGGGAGCCCTGGTCACCGTCTCCTCAGC.
Nucleotide sequence shown in SEQ ID NO: 4 
GTACATTCAGAAATTGTGTTGACGCAGTCTCCAGGCACCCTGTCTTTGTCTCCAG
GGGAAAGAGCCACCCTCTCCTGCAGGGCCAGTCAGAGTGTTACCAGCAGCCAGTTA
 
GCCTGGTACCAGCAGAAACCTGGCCAGGCTCCCAGACTCCTCATCTATGGTGAATCC
AGCAGGGCCACTGGCATCCCAGACAGGTTCAGTGGCAGGGGGTCTGGGACAGACTT
CGCTCTCACCATCAGCAGACTGGAGCCTGAAGATTTTGCAGTGTATTGCTGTCAGCA
GTATGGTACTTCAATCGCTTTCGGCGGAGGGACCAAGGTGGAGATCAAA.
SEQ ID NO: 5 (amino acid sequence of human TSHR)













.

**Table 1 CDRs of a heavy chain (H) and a light chain (L) of the fully human TSHR-blocking monoclonal antibody TBI80**

| No. | H-CDR1 | H-CDR2 | H-CDR3 | L-CDR1 | L-CDR2 | L-CDR3 |
|---|---|---|---|---|---|---|
| TBI8 0 | QYSMN (SEQ ID NO: 6) | YISSSAATVFYADSV QG (SEQ ID NO: 7) | EAVIVDGMPFE Y (SEQ ID NO: 8) | RASQSVTSS QLA (SEQ ID NO: 9) | GESSRA T (SEQ ID NO: 10) | QQYGTSIA (SEQ ID NO: 11) |
| Kl-70 | DNWIG | IIYPGDSDTRYSPSF QG | LDWNYNPLRY | SGSSSDIGSN YVS | DNNKR PS | GTWDSRLGI AV |

The nucleotide sequence described in the present disclosure or at least a portion thereof can be expressed by an appropriate expression system to obtain the corresponding protein or polypeptide. The expression system includes, but is not limited to, bacterial, insect cell, and mammalian cell expression systems.

The present disclosure also provides an expression vector including the nucleotide sequence encoding the fully human TSHR-blocking monoclonal antibody.

The present disclosure also provides a host cell including the nucleotide sequence or the expression vector. Preferably, the host cell is a CHO-K1 cell, etc.

The present disclosure also provides a cell, where the cell is an isolated cell including the fully human TSHR-blocking monoclonal antibody described above and/or the nucleotide sequence described above and/or the expression vector described above.

The present disclosure also provides a cell, where the cell is an isolated cell expressing the fully human TSHR-blocking monoclonal antibody described above.

The present disclosure also provides a cell, where the cell is an isolated cell secreting the fully human TSHR-blocking monoclonal antibody described above.

The present disclosure also provides a composition including a determined concentration of a TSHR autoantibody and the fully human TSHR-blocking monoclonal antibody described above.

The present disclosure also provides a pharmaceutical composition for administering to a mammalian subject to treat a thyroid-related disorder, where the pharmaceutical composition includes the fully human TSHR-blocking monoclonal antibody described above and a pharmaceutically acceptable carrier.

The thyroid-related disorder is selected from hyperthyroidism, thyroid-associated ophthalmopathy (Graves' ophthalmopathy), neonatal hyperthyroidism, human chorionic gonadotropin-induced hyperthyroidism, excessive thyroid activity, thyroid cancer, thyroiditis, and pretibial myxedema.

Further, the expression vector may include a plasmid, a virus, or a fragment thereof, and various different types of vectors known to those skilled in the art.

Specifically, the pharmaceutical composition may include a determined concentration of a TSHR autoantibody with a TSH antagonist activity, and the fully human TSHR-blocking monoclonal antibody according to the present disclosure.

Further, the pharmaceutical composition is suitable for administration to a human. Preferably, the pharmaceutical composition according to the present disclosure has no significant adverse effect on an immune system of a subject.

Further, the pharmaceutical composition includes one or more additional TSHR antagonists.

Further, the pharmaceutical composition is for use in treating a thyroid-related disorder in an injectable form.

Preferably, the pharmaceutical composition is for use in treating Graves' ophthalmopathy in a form of an intravenous injection or an eye drop.

Preferably, the pharmaceutical composition is for use in treating pretibial myxedema in a topical administration form.

Specifically, the pharmaceutical composition includes any fully human TSHR-blocking monoclonal antibody according to the present disclosure and any pharmaceutically acceptable carrier, adjuvant, or vehicle. The pharmaceutically acceptable carrier, adjuvant, or vehicle that can be used in the pharmaceutical composition of the present disclosure includes, but is not limited to, a buffer substance (such as a phosphate), glycine, an ion exchanger, alumina, aluminum stearate, lecithin, a serum protein (such as human serum albumin), sorbic acid, potassium sorbate, water, sodium chloride, a salt or an electrolyte (such as protamine sulfate), disodium hydrogen phosphate, dipotassium hydrogen phosphate, a partial glyceride mixture of saturated plant fatty acids, a zinc salt, colloidal silica, a cellulose-based substance, polyethylene glycol, sodium carboxymethylcellulose, polyacrylate, a wax, a polyethylene-polyoxypropylene block polymer, magnesium trisilicate, polyvinylpyrrolidone, polyethylene glycol, and lanolin.

A dosage form of the pharmaceutical composition includes a capsule, a tablet, an aqueous suspension, a solution, a rectal suppository, an enema, an ointment, a lotion, a cream, a nasal spray, and an inhalant. Preferably, the dosage form of the pharmaceutical composition is a solution or an ointment.

Specifically, the pharmaceutical composition may be a sterile injectable formulation, such as a sterile injectable oil suspension or aqueous suspension. Such a suspension can be prepared from a suitable dispersing or wetting agent (such as Tween 80) and a suspending agent according to a technique known in the art. The sterile injectable formulation may also be a sterile injectable solution or suspension prepared with a non-toxic parenterally acceptable diluent or solvent, such as a solution in 1,3-butanediol. Available acceptable vehicles and solvents include water, mannitol, a Ringer's solution, and an isotonic sodium chloride solution. In addition, sterile fixed oils are commonly used as a solvent or a suspending medium. For this purpose, any mild fixed oil can be employed, including synthetic monoglycerides or diglycerides. Fatty acids (such as oleic acid and glyceride derivatives thereof) can be used in preparation of injections. The oil solution or suspension may also include a long-chain alcohol diluent or dispersing agent.

Specifically, an administration route for the pharmaceutical composition may include topical administration, administration by spray inhalation, oral administration, parenteral administration, administration by an eye drop or eye ointment, buccal administration, vaginal administration, rectal administration, nasal administration, administration by an implantable reservoir, etc. Preferably, the administration route for the pharmaceutical composition is oral administration or injection. The term "parenteral" as used in the present disclosure includes subcutaneous, intradermal, intra-synovial, intrasternal, intravenous, intramuscular, intralesional, intracranial, intra-articular, and intrathecal injection or infusion techniques.

Specifically, the pharmaceutical composition may also be administered as a suppository for rectal administration. The pharmaceutical composition can be prepared by mixing the active ingredient of the present disclosure with a suitable non-irritating excipient. The excipient is a solid at room temperature but a liquid at rectal temperature, and thus can melt in the rectum to release the active ingredient. The excipient includes, but is not limited to, beeswax, cocoa butter, and polyethylene glycol.

Specifically, the pharmaceutical composition can be administered orally in any orally acceptable dose form, including, but not limited to, a tablet, a capsule, an aqueous suspension, and a solution. For oral tablets, common carriers include corn starch and lactose. A lubricant, such as magnesium stearate, is usually further added. For oral capsules, useful diluents include lactose and dried corn starch. When an aqueous suspension is administered orally, an active ingredient of the aqueous suspension is combined with an emulsifying agent and a suspending agent. If desired, some flavoring agents and/or sweeteners and/or coloring agents may also be added.

Specifically, the pharmaceutical composition may be administered in a form of a nasal spray or an inhalant. The pharmaceutical composition is prepared according to techniques well known in the field of pharmaceutical formulations, and can be prepared into a saline solution using benzyl alcohol or other suitable preservatives, an absorption enhancer for enhancing bioavailability, a fluorocarbon, and/or other dispersing agents or solubilizers known in the art.

Specifically, when a desired treatment involves an area or organ that is easily accessible by topical application, topical administration of the pharmaceutical composition of the present disclosure is particularly useful. For topical skin administration, the pharmaceutical composition should be prepared into a suitable ointment including the active ingredient suspended or dissolved in a carrier. A carrier for the topical administration of the pharmaceutical composition of the present disclosure includes, but is not limited to, mineral oil, white petrolatum, propylene glycol, liquid petroleum, emulsifying wax, a polyoxyethylene-polyoxypropylene compound, and water. Alternatively, the pharmaceutical composition may be prepared into a suitable cream or lotion including the active ingredient suspended or dissolved in a carrier. Suitable carriers include, but are not limited to, mineral oil, polysorbate 60, cetyl esters wax, cetearyl alcohol, sorbitan monostearate, benzyl alcohol, 2-octyldodecanol, and water. The pharmaceutical composition of the present disclosure may also be administered to a lower intestinal tract in a form of a rectal suppository or a suitable enema. The present disclosure also includes a topical transdermal patch.

The present disclosure also provides a use of the pharmaceutical composition in preparation of a drug for treating a disease such as hyperthyroidism or thyroid-associated ophthalmopathy; in preparation of a drug for inhibiting thyroid hyperplasia and/or thyroid hormone production; in preparation of a drug for blocking TSHR; or in preparation of a drug for antagonizing an activating effect of TSH on TSHR.

In the method of the present disclosure, no animal immunization is required, but antibody genes are directly amplified from human single B cells. The method is specifically as follows: Antibody genes are directly amplified from isolated single plasma cells or memory B cells to obtain a large number of naturally paired antibody genes for heavy and light chains. These paired antibody genes for heavy and light chains are then expressed, and antibodies with antigen specificity and neutralizing activity are ultimately identified by screening. The method of the present disclosure offers advantages such as high speed, high throughput, and small cell consumption. The fully human antibody prepared accordingly retains the excellent diversity of genes and the natural pairing of heavy-chain and light-chain variable regions, presenting tremendous superiority. Compared with conventional hybridoma antibody preparation technology, the method of the present disclosure can significantly shorten the experimental timeline. The conventional hybridoma antibody preparation technology requires approximately 3 months to acquire an antibody sequence. However, it takes only 3 weeks to 1 month for the method of the present disclosure to acquire an antibody sequence, which greatly reduces the workload and cost of antibody preparation.

The present disclosure further provides the following beneficial effects: The fully human TSHR-blocking monoclonal antibody of the present disclosure can specifically bind to TSHR and exhibits excellent inhibitory activity in both *in vitro* and *in vivo* experiments. Therefore, the fully human TSHR-blocking monoclonal antibody demonstrates promising application prospects in the development of treatments for GD. There are numerous GD patients worldwide, but the current treatment options for GD have many limitations. The fully human TSHR-blocking monoclonal antibody prepared by the present disclosure can effectively block the binding of TSH to TSHR to inhibit thyroid hyperplasia and/or thyroid hormone production without inducing a human antimouse antibody response and many side effects. Consequently, the fully human TSHR-blocking monoclonal antibody holds broad application prospects.

### DESCRIPTION OF THE DRAWINGS

To describe the technical solutions in the embodiments of the present disclosure or in the prior art clearly, the accompanying drawings required for describing the embodiments or the prior art are briefly described below. Apparently, the accompanying drawings in the following description show merely some embodiments of the present disclosure, and those skilled in the art may still derive other drawings from these accompanying drawings without creative efforts.
FIG. 1 shows the sorting of plasma cells and memory single B cells that specifically recognize TSHR from peripheral blood by flow cytometry in the present disclosure, where a Biotin-AF647-labeled TSHR protein is used as a bait; other antibodies used for the sorting are as follows: CD19-Pacific Blue, IgM-PE, CD27-BV605, and CD38-PE-Cy7; and TSHR-specific plasma cells and memory single B cells finally isolated are designated as CD19+IgM-CD27+CD38-TSHR+;
FIG. 2 shows the screening of the anti-human TSHR monoclonal antibodies of the present disclosure in terms of an antigen-binding capacity;
FIG. 3 shows the screening of the anti-human TSHR monoclonal antibodies of the present disclosure in terms of a blocking activity, where results are expressed as mean ± standard deviation (n = 3);
FIG. 4 shows sodium dodecyl sulfate-polyacrylamide gel electrophoresis (SDS-PAGE) results for the *in vitro* expression of the antibody TBI80 according to the present disclosure, where R represents a reduced state and NR represents a non-reduced state;
FIG. 5 shows *in vitro* dilution blocking activity assay results of the antibody TBI80 according to the present disclosure, where bTSH is added at an amount of 5 ng/mL and hTSH is added at an amount of 100 ng/mL;
FIG. 6 shows changes of T4 and TSH levels in rats intramuscularly injected with the antibody TBI80 of the present disclosure at different time points, where results are expressed as mean ± standard deviation (n = 5) and **p* < 0.05; and
FIG. 7 shows therapeutic outcomes of the antibody TBI80 of the present disclosure in a GD mouse model after three administrations, where A shows gross morphologies of thyroids after a treatment; B shows thyroid weights; C shows changes of T4 levels *in vivo;* D shows *in situ* gross morphologies of thyroids; E shows histomorphologies of thyroids by Hematoxylin and Eosin (HE) staining, with a scale bar of 100 µm; and results are expressed as mean ± standard deviation (n = 5 to 6), **p* < 0.05, and ***p* < 0.01.

### SPECIFIC IMPLEMENTATIONS

The present disclosure will be further described in detail with reference to the following specific examples and accompanying drawings. The process, conditions, and experimental methods for implementing the present disclosure, except for the content specially mentioned below, are all common general knowledge in the art. The present disclosure imposes no special limitation on the content.

### Example 1

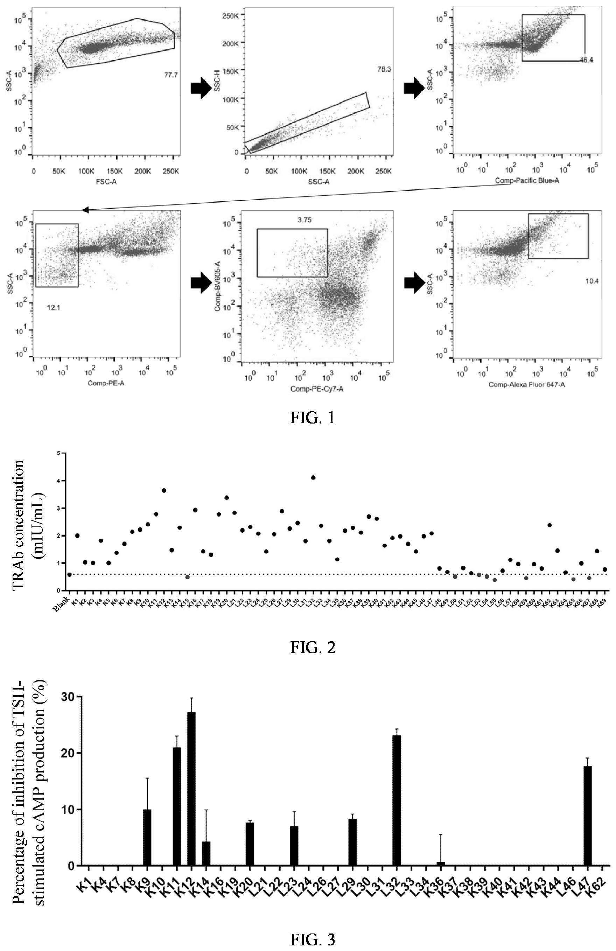

Single-cell sorting: Peripheral blood was collected from a volunteer with a high TBAb titer. A human B cell enrichment mixture for immunodensity gradient centrifugation (STEMCELL, Cat. No. 15024) was added to the peripheral blood, and density gradient centrifugation was conducted using Histopaque^{®}-1077 (Sigma, Cat. No. 10771) to obtain B cells from the peripheral blood. CD19⁺IgM⁻CD27⁺CD38⁻TSHR⁺ plasma cells and memory single B cells were isolated by flow cytometry sorting (FIG. 1).

Cloning of genes for heavy-chain and light-chain variable regions: RNA was concentrated and purified from single cell lysate using SPRlselect nucleic acid fragment selection kit (Beckman Coulter, Cat. No. B23317), and cDNA was synthesized according to instructions of a SuperScript^{™} IV one-step reverse transcription-polymerase chain reaction (RT-PCR) system (Invitrogen, Cat. No. 12594100). With the cDNA as a template, antibody genes for heavy-chain and light-chain variable regions were amplified by PCR using DreamTaq Green PCR 2X Master Mix (ThermoFisher, Cat. No. K1081). Amplified PCR products were then subjected to agarose gel electrophoresis. Bands with expected fragment sizes were cut and recovered. DNA fragments were purified using QIAquick Gel Extraction Kit (QIAGEN, Cat. No. 28704) and sent for sequencing. Sequencing results were analyzed using the IgBLAST function of the National Center for Biotechnology Information (NCBI) or the IMGT database, and cloning primers corresponding to V and J genes were selected for cloning PCR of antibody genes for heavy and light chains. Then, fragment ligation was conducted using NEBuilder^{®} HiFi DNA Assembly Master Mix (NEB, Cat. No. E2621L). Gene sequences for the heavy-chain and light-chain variable regions were cloned respectively into heavy-chain and light-chain expression vectors (NCBI GenBank Accession Nos. FJ475055, FJ475056, and FJ517647) including respective constant regions of heavy and light chains. Transformation and plating were conducted. Colonies were picked and sequenced to determine a final sequence.

### Screening of blocking antibodies

1. Screening based on an antigen-binding capacity: Heavy-chain and light-chain expression vectors were co-transfected into 293T cells at a ratio of 1:1 and cultured for 3 d at 37°C and 5% CO₂. Then, a culture supernatant was collected by centrifugation, and tested for a TRAb titer according to instructions of a human anti-TSHR antibody enzyme-linked immunosorbent assay kit (ELK Biotechnology CO., LTD., Cat. No. ELK9540). Accordingly, heavy and light chain combinations with a lower antigen-binding capacity than a blank control group were removed. The remaining combinations were screened based on a blocking activity (FIG. 2).
2. Screening based on a blocking activity: Heavy-chain and light-chain expression vectors were co-transfected into 293T cells at a ratio of 1:1 and cultured for 3 d at 37°C and 5% CO₂. Then, a culture supernatant was collected by centrifugation. 100 µL of the culture supernatant was taken, and 1 IU/L bTSH (Sigma) was added. A resulting mixture was incubated with hTSHR-CHO cells for 2 h. A cell lysate was prepared and tested for a change in a cAMP level (R&D, Cat. No. KGE002B). Accordingly, antibody combinations with a strong blocking activity were retained for further validation (FIG. 3). Among the retained antibody combinations, a combination K12 exhibited the strongest inhibitory activity, and a corresponding monoclonal antibody was designated TBI80 and would be further expressed and purified subsequently.

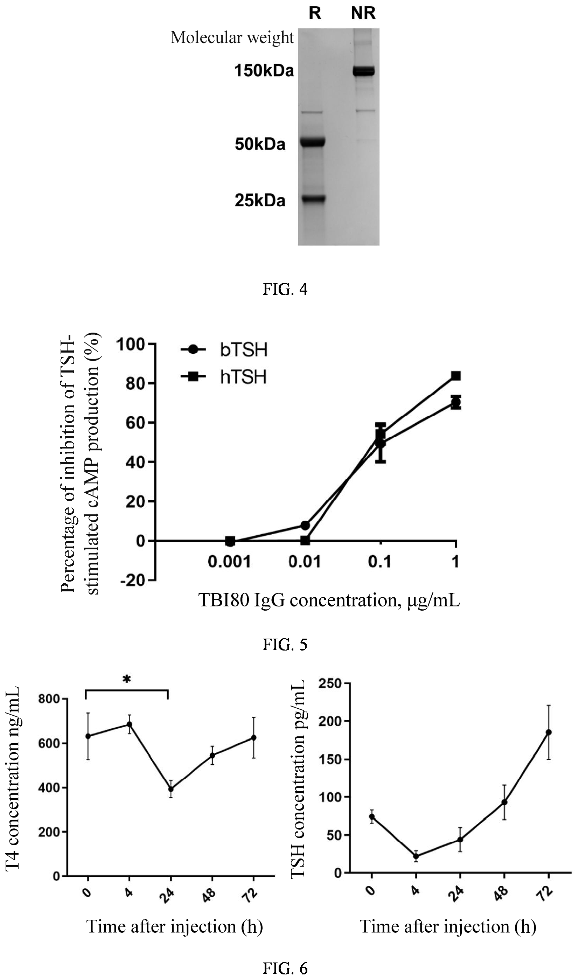

Antibody expression and purification: Heavy-chain and light-chain expression vectors were co-transfected into 293T cells at a ratio of 1:1 and cultured for 5 d at 37°C and 8% CO₂ under 130 rpm shaking. A culture supernatant was collected by centrifugation, filtered through a 0.45 µm filter, and purified by affinity chromatography using Protein A (GenScript, Cat. No. L00210) to obtain an antibody protein with a high purity. An antibody concentration was determined by the Bradford protein assay (Beyotime, Cat. No. P0006) and the NanoDrop A280 method. The antibody expression was detected by SDS-PAGE and Coomassie Brilliant Blue staining (Beyotime, Cat. No. P0017F) (FIG. 4).

*In vitro* efficacy evaluation: The purified monoclonal antibody was serially diluted to different concentrations, and 5 ng/mL bTSH or 100 ng/mL human TSH was added. Resulting mixtures were each co-incubated with hTSHR-CHO cells for 2 h. Cell lysates were prepared and tested for changes in cAMP levels (FIG. 5). It was found that the monoclonal antibody TBI80 could effectively antagonize an activating effect of TSH on TSHR at a concentration of 1 µg/mL, with concentration dependence.

*In vivo* efficacy evaluation: Male SD rats aged 6 weeks to 8 weeks were selected. Each rat was intramuscularly injected with 0.1 mL of a TBI80 monoclonal antibody solution (which included 200 µg of the monoclonal antibody TBI80; employed the following phosphate-buffered saline (PBS): 137 mmol/L sodium chloride, 2.7 mmol/L potassium chloride, 8.1 mmol/L disodium hydrogen phosphate, 1.5 mmol/L potassium dihydrogen phosphate, pH 7.4; and included 3% mannitol and 1% Tween-80). Blood was collected from a tail vein at 0 h before injection and at 4 h, 24 h, 48 h, and 72 h after injection. Serum was isolated by centrifugation at 4,000 rpm and 4°C for 5 min and properly stored at -80°C. Concentrations of thyroxine T4 (Cloud-Clone, Cat. No. CEA452Ge) and TSH (Cloud-Clone, Cat. No. CEA463Ra) were determined (FIG. 6). Compared with test results at 0 h before injection, a T4 level significantly decreased at 24 h after injection, indicating that TBI80 still exhibited inhibitory activity *in vivo.* Subsequently, due to the feedback elevation of TSH, the T4 level partially recovered.

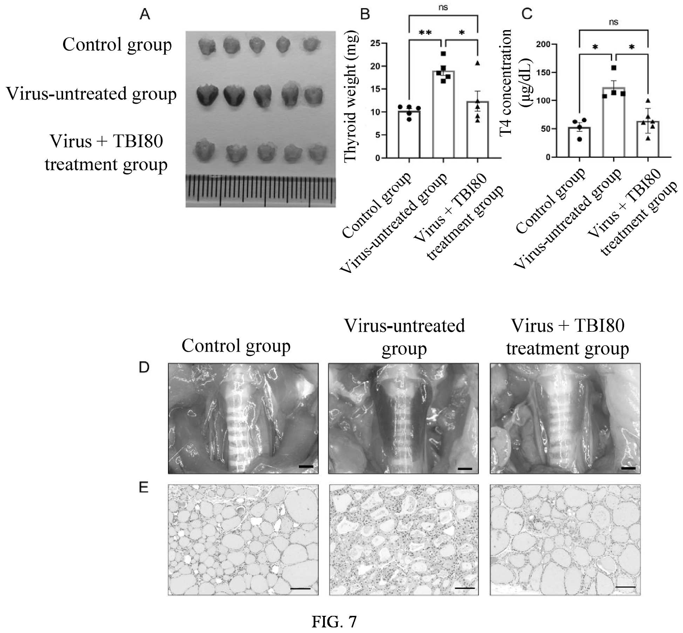

Establishment of a GD mouse model: BALB/c mice were intramuscularly injected with an adenovirus overexpressing an α subunit of human TSHR (Ad-TSHR289) three times at an interval of 3 weeks. GD model evaluation was conducted 3 weeks after the final immunization. Mice were then grouped (a control group, a virus-untreated group, and a virus + TBI80 treatment group) and received corresponding treatments. 3 weeks later, the treatments were repeated. Blood samples were collected (for detection of thyroid function, liver and kidney function, etc.), and changes in thyroid size, weight, and morphology were observed (FIG. 7). Compared with the untreated group, after three times of a 50 µg TBI80 treatment, a T4 level was significantly declined, a thyroid size essentially returned to a normal level, a thyroid weight and morphology were improved, and thyroid follicles were regular with only a few epithelial cells remaining tall columnar. These results further indicated that the TSHR-blocking monoclonal antibody TBI80 had *in vivo* inhibitory activity.

The protection content of the present disclosure is not limited to the above example. Changes and advantages that can be easily figured out by those skilled in the art without departing from the spirit and scope of the present disclosure are included in the present disclosure and subject to the protection scope defined by the appended claims.

## Claims

1. A fully human thyroid-stimulating hormone receptor (TSHR)-blocking monoclonal antibody, wherein the antibody is capable of binding to TSHR to block binding of thyroid-stimulating hormone (TSH) to the TSHR; the amino acid sequence of the antibody is shown in SEQ ID NO: 1 or 2; and the nucleotide sequence for the antibody is shown in SEQ ID NO: 3 or 4.

2. The antibody according to claim 1, wherein an amino acid sequence of the antibody has a sequence identity of at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% with SEQ ID NO: 1 or 2; and/or a nucleotide sequence for the antibody has a sequence identity of at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% with SEQ ID NO: 3 or 4.

3. The fully human TSHR-blocking monoclonal antibody according to claim 1 or 2, wherein the antibody is an antagonist against the TSH and/or an antagonist against a TSHR-stimulating antibody.

4. The fully human TSHR-blocking monoclonal antibody according to claim 1 or 2, wherein the antibody comprises a V_{H} region, and the V_{H} region comprises one or more complementarity determining regions (CDRs) selected from SEQ ID NO: 6 to SEQ ID NO: 8; and/or
the antibody comprises a V_{L} region, and the V_{L} region comprises one or more CDRs selected from SEQ ID NO: 9 to SEQ ID NO: 11; and/or
the antibody comprises one or more amino acid sequences substantially homologous to CDRs.

5. A substance, comprising:
(1) a formulation, a drug, or a pharmaceutical composition that comprises the fully human TSHR-blocking monoclonal antibody according to any one of claims 1 to 2;
(2) a reagent or a kit that comprises the fully human TSHR-blocking monoclonal antibody according to claim 1 or 2;
(3) a nucleotide sequence encoding the fully human TSHR-blocking monoclonal antibody according to claim 1 or 2, wherein the nucleotide sequence is one of the following sequences:
(a) the nucleotide sequence shown in SEQ ID NO: 3 or SEQ ID NO: 4;
(b) a nucleotide sequence having the sequence identity of at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% with SEQ ID NO: 3 or SEQ ID NO: 4;
(c) a nucleotide sequence obtained by adding, substituting, deleting, or inserting one or more nucleotides relative to the nucleotide sequence shown in SEQ ID NO: 3 or SEQ ID NO: 4;
(d) a nucleotide sequence capable of hybridizing to the nucleotide sequence defined in the (a), the (b), or the (c) or a full-length complement thereof under stringent conditions; or
(e) a nucleotide sequence different from the nucleotide sequence defined in the (a), the (b), the (c), or the (d) due to genetic code degeneracy,
wherein the nucleotide sequence or a portion thereof encodes an antibody V_{H} domain and an antibody V_{L} domain or the CDRs selected from SEQ ID NO: 6 to SEQ ID NO: 11;
(4) an expression vector comprising the nucleotide sequence defined in the (3);
(5) a host cell comprising the nucleotide sequence defined in the (3) and/or the expression vector defined in the (4);
(6) a cell, wherein the cell is an isolated cell comprising the fully human TSHR-blocking monoclonal antibody according to claim 1 or 2 and/or the nucleotide sequence defined in the (3) and/or the vector defined in the (4); and/or
an isolated cell expressing the fully human TSHR-blocking monoclonal antibody according to claim 1 or 2; and/or
an isolated cell secreting the fully human TSHR-blocking monoclonal antibody according to claim 1 or 2;
(7) a composition comprising a determined concentration of a TSHR autoantibody and the fully human TSHR-blocking monoclonal antibody according to claim 1 or 2; or
(8) a pharmaceutical composition for administering to a mammalian subject to treat a thyroid-related disorder, wherein the pharmaceutical composition comprises the fully human TSHR-blocking monoclonal antibody according to claim 1 or 2 and a pharmaceutically acceptable carrier; and
the thyroid-related disorder is selected from hyperthyroidism, thyroid-associated ophthalmopathy, neonatal hyperthyroidism, human chorionic gonadotropin-induced hyperthyroidism, excessive thyroid activity, thyroid cancer, thyroiditis, and pretibial myxedema.

6. The substance according to claim 5, wherein the pharmaceutical composition is suitable for administration to a human.

7. The substance according to claim 5, wherein the pharmaceutical composition comprises one or more additional TSHR antagonists.

8. The substance according to claim 5, wherein the pharmaceutical composition comprises the fully human TSHR-blocking monoclonal antibody according to claim 1 or 2 and any pharmaceutically acceptable carrier, adjuvant, or vehicle.

9. The substance according to claim 5, wherein an administration route for the pharmaceutical composition comprises oral administration, parenteral administration, administration by spray inhalation, topical administration, administration by an eye drop or eye ointment, rectal administration, nasal administration, buccal administration, vaginal administration, and administration by an implantable reservoir.

10. The substance according to claim 5, wherein a dosage form of the pharmaceutical composition comprises a capsule, a tablet, an aqueous suspension, a solution, a rectal suppository, an enema, an ointment, a lotion, a cream, a nasal spray, and an inhalant.

11. The substance according to claim 5, wherein the pharmaceutical composition is for use in treating the thyroid-related disorder in an injectable form.

12. The substance according to claim 5, wherein the pharmaceutical composition is for use in treating the pretibial myxedema in a topical administration form; and/or
the pharmaceutical composition is for use in treating Graves' ophthalmopathy in a form of an intravenous injection or an eye drop.

13. A method, comprising:
(1) a method for preparing the fully human TSHR-blocking monoclonal antibody, specifically comprising the following steps:
step 1, isolating plasma cells and memory B cells targeting the TSHR from peripheral blood of a patient with a high TSHR-blocking antibody (TBAb) activity; conducting single-cell RNA extraction and cDNA synthesis, and verifying genes of heavy chains H, a light chain λ, and a light chain κ amplified from isolated single cells by nested polymerase chain reaction (PCR); and selecting single-cell clones positive for both heavy chains and light chains for subsequent cloning;
step 2, conducting *in vitro* amplification by the nested PCR, and cloning genes of B cell receptor (BCR) heavy and light chains from all single B cells into a heavy-chain expression vector AbVec-IGHG1, a light-chain λ expression vector AbVec-hlgKappa, and a light-chain λ expression vector AbVec-hIgLambda, respectively; and
step 3, after heavy-chain and light-chain recombinant plasmids are successfully obtained, subjecting acquired candidate clones to sequencing and alignment analysis to determine numbers of nucleotide and amino acid sequences of candidate antibodies; transfecting the heavy-chain and light-chain recombinant plasmids to express monoclonal antibodies *in vitro;* and further verifying an antigen-binding capacity and an antibody blocking activity to finally obtain an antibody combination specifically targeting a target antigen, which is the fully human TSHR-blocking monoclonal antibody;
(2) a method for treating a thyroid-related disorder in a mammalian subject or in a cell derived from the subject, comprising contacting the subject or the cell with the fully human TSHR-blocking monoclonal antibody according to claim 1 or 2;
(3) a method for inhibiting stimulation of the TSHR by a TSHR-stimulating antibody in a thyroid of a mammalian subject, comprising contacting the subject with the antibody of the present disclosure;
(4) a method for inhibiting binding of a TSHR-stimulating autoantibody to extrathyroidal TSHR in a mammalian subject, comprising contacting the subject with the fully human TSHR-blocking monoclonal antibody according to claim 1 or 2;
(5) a method for treating thyroid cancer or metastatic thyroid cancer in a thyroid of a subject or in a thyroid cell derived from the subject, comprising contacting the cancer cell with the fully human TSHR-blocking monoclonal antibody according to claim 1 or 2 to inhibit a constitutive TSHR activity in the cell;
(6) a method for treating excessive thyroid activity caused by a constitutive thyroid activity in a subject or in a thyroid cell derived from the subject, comprising contacting the subject or the cell with the fully human TSHR-blocking monoclonal antibody according to claim 1 or 2 to inhibit the excessive thyroid activity;
(7) a method for identifying a molecule capable of inhibiting binding of the TSHR-stimulating antibody to the TSHR, comprising providing at least one fully human TSHR-blocking monoclonal antibody according to claim 1 or 2 as a reference; or
(8) a method for identifying a molecule capable of inhibiting binding of TBAb to the TSHR, comprising providing at least one fully human TSHR-blocking monoclonal antibody according to claim 1 or 2 as a reference.

14. The method according to claim 13, wherein the thyroid-related disorder is selected from hyperthyroidism, thyroid-associated ophthalmopathy, neonatal hyperthyroidism, human chorionic gonadotropin-induced hyperthyroidism, excessive thyroid activity, thyroid cancer, thyroiditis, and pretibial myxedema.

15. The method according to claim 13, wherein the subject is a human.

16. The method according to claim 13, wherein in the method (3), the TSHR-stimulating antibody is prevented from binding to the TSHR.

17. The method according to claim 13, wherein in the method (4), the extrathyroidal TSHR is located in a retro-orbital tissue and/or a pretibial tissue of the subject; and/or
the antibody blocks the binding of the TSHR-stimulating autoantibody to the extrathyroidal TSHR.

18. The method according to claim 13, wherein in the method (5), regrowth of a thyroid cancer cell is prevented or delayed.

19. The method according to claim 13, wherein in the method (7), a test molecule capable of blocking the binding of the TSHR-stimulating antibody to the TSHR is selected.

20. The method according to claim 13, wherein in the method (8), a molecule capable of blocking the binding of the TBAb to the TSHR is selected.

21. A use, comprising:
(1) a use of the fully human TSHR-blocking monoclonal antibody according to claim 1 or 2 in treating a thyroid-related disorder;
(2) a use of the fully human TSHR-blocking monoclonal antibody according to claim 1 or 2 in preparation of a drug for treating the thyroid-related disorder;
(3) a use of the fully human TSHR-blocking monoclonal antibody according to claim 1 or 2 in preparation of a reagent or a kit for detecting a TSHR antibody;
(4) a use of the fully human TSHR-blocking monoclonal antibody according to claim 1 or 2 in preparation of a reagent or a kit or a product for detecting hyperthyroidism or thyroid-associated ophthalmopathy; or
(5) a use of the fully human TSHR-blocking monoclonal antibody according to claim 1 or 2 or a substance according to claim 5 or a method according to claim 13 in preparation of a formulation, a drug, or a pharmaceutical composition for detecting the TSHR antibody; in preparation of a drug for treating the hyperthyroidism or the thyroid-associated ophthalmopathy; in preparation of a drug for inhibiting thyroid hyperplasia and/or thyroid hormone production; in preparation of a drug for blocking the TSHR; or in preparation of a drug for antagonizing an activating effect of the TSH on the TSHR.

22. The use according to claim 21, wherein the thyroid-related disorder is selected from the hyperthyroidism, the thyroid-associated ophthalmopathy, neonatal hyperthyroidism, human chorionic gonadotropin-induced hyperthyroidism, excessive thyroid activity, thyroid cancer, thyroiditis, and pretibial myxedema.
